# EUROPEAN PATENT APPLICATION

(11) **EP 4 703 007 A1**
(43) Date of publication of application: **04.03.2026**
(21) Application number: 24197943.4
(22) Date of filing: 02.09.2024
(51) Int. Cl.: A61N 5/06

(54) **SYSTEM FOR TRAINING USERS IN THE OPERATION OF A SKIN CARE DEVICE**

(71) Applicant: Koninklijke Philips N.V., 5656 AG Eindhoven (NL)
(72) Inventor: KURTCEBE, Bayram Yavuz, Eindhoven (NL)
(74) Representative: Philips Intellectual Property & Standards

(57) **Abstract**

A light-based skin care device is disclosed, comprising a light source capable of operating in multiple light intensity modes and a processor for selecting a light intensity mode. The device is characterized by a training mode that allows users to practice operation without causing harm. This training mode operates at a light intensity level below a predefined threshold, ensuring safety during user training.

## Description

### FIELD OF THE INVENTION

The present disclosure relates to skin treatment devices, in particular to systems and methods for training users in the operation of these devices.

### BACKGROUND OF THE INVENTION

Devices for skin treatments that utilize light-based technology are prevalent in the consumer market, offering a range of applications from hair removal to skin rejuvenation. These devices require users to maneuver them over the skin with precision due to the specific size of the active area relative to the device's tip. Inexperienced users may find the estimation of the active area's dimensions challenging, which can lead to inconsistent coverage and suboptimal treatment results. The need for a more intuitive guidance system is evident, one that would assist users in achieving comprehensive and effective treatment coverage.

Additionally, the preparatory phase for using these light-based skin treatment devices typically involves a sensitivity test. Users apply the device to various skin areas at different light intensities and monitor for reactions, a time consuming process. An opportunity exists to enhance user experience during this initial phase by providing a means to practice device handling and operation. A solution that offers a risk-free, interactive learning experience during this period would be beneficial, enabling users to develop proper technique and thereby improving the overall effectiveness of the treatment.

### SUMMARY OF THE INVENTION

Particular and preferred aspects of the invention are set out in the accompanying independent and dependent claims. Features from the dependent claims may be combined with features of the independent claims and with features of other dependent claims as appropriate and not merely as explicitly set out in the claims.

These and other aspects of the invention will be apparent from and elucidated with reference to the embodiment(s) described hereinafter.

In an aspect of the invention, a light-based skin care device is presented, comprising a light source for treating skin, the light source configured to operate in a plurality light intensity modes; a processor configured for selecting a light intensity mode of the light source; characterized in that: at least one light intensity mode of the plurality light intensity modes is a training mode for allowing users to practice operation of the system without causing harm to a user.

According to embodiments, the device comprises an IMU (Inertial Measurement Unit) sensor for providing movement information of the device, and wherein the training mode instructs the user on how to move the device for effective skin treatment.

According to embodiments, the training mode instructs the user on how often to activate the device for effective treatment.

According to embodiments, the processor is configured to select the training mode during first time use by the user.

According to embodiments, the training mode is selectable via a user interface integrated in a software application running on a device external from the light based skin care device.

According to embodiments, the light based skin care device is an IPL (Intense Pulsed Light) device.

According to embodiments, the at least one light intensity mode is a light intensity level below a predefined threshold light intensity level.

According to embodiments, the predefined threshold light intensity level is equal0.2 J/cm^2.

### BRIEF DESCRIPTION OF THE DRAWINGS

The drawings are only schematic and are non-limiting. In the drawings, the size of some of the elements may be exaggerated and not drawn on scale for illustrative purposes.

Any reference signs in the claims shall not be construed as limiting the scope.

In the different drawings, the same reference signs refer to the same or analogous elements.
FIG 1. illustrates a box diagram of a light-based skin care device
FIG 2. illustrates a box diagram of a light-based skin care device including a user interface on the device itself
FIG 3. illustrates an box diagram of a light-based skin care device, a smartphone is in wireless communication with the device. The smartphone includes the user interface.

### DETAILED DESCRIPTION OF THE EMBODIMENTS

The present invention will be described with respect to particular embodiments and with reference to certain drawings but the invention is not limited thereto but only by the claims. The drawings described are only schematic and are non-limiting. In the drawings, the size of some of the elements may be exaggerated and not drawn on scale for illustrative purposes. The dimensions and the relative dimensions do not correspond to actual reductions to practice of the invention.

Furthermore, the terms first, second and the like in the description and in the claims, are used for distinguishing between similar elements and not necessarily for describing a sequence, either temporally, spatially, in ranking or in any other manner. It is to be understood that the terms so used are interchangeable under appropriate circumstances and that the embodiments of the invention described herein are capable of operation in other sequences than described or illustrated herein.

It is to be noticed that the term "comprising", used in the claims, should not be interpreted as being restricted to the means listed thereafter; it does not exclude other elements or steps. It is thus to be interpreted as specifying the presence of the stated features, integers, steps or components as referred to, but does not preclude the presence or addition of one or more other features, integers, steps or components, or groups thereof. Thus, the scope of the expression "a device comprising means A and B" should not be limited to devices consisting only of components A and B. It means that with respect to the present invention, the only relevant components of the device are A and B.

Reference throughout this specification to "one embodiment" or "an embodiment" means that a particular feature, structure or characteristic described in connection with the embodiment is included in at least one embodiment of the present invention. Thus, appearances of the phrases "in one embodiment" or "in an embodiment" in various places throughout this specification are not necessarily all referring to the same embodiment, but may. Furthermore, the particular features, structures or characteristics may be combined in any suitable manner, as would be apparent to one of ordinary skill in the art from this disclosure, in one or more embodiments.

Similarly it should be appreciated that in the description of exemplary embodiments of the invention, various features of the invention are sometimes grouped together in a single embodiment, figure, or description thereof for the purpose of streamlining the disclosure and aiding in the understanding of one or more of the various inventive aspects. This method of disclosure, however, is not to be interpreted as reflecting an intention that the claimed invention requires more features than are expressly recited in each claim. Rather, as the following claims reflect, inventive aspects lie in less than all features of a single foregoing disclosed embodiment. Thus, the claims following the detailed description are hereby expressly incorporated into this detailed description, with each claim standing on its own as a separate embodiment of this invention.

Furthermore, while some embodiments described herein include some but not other features included in other embodiments, combinations of features of different embodiments are meant to be within the scope of the invention, and form different embodiments, as would be understood by those in the art. For example, in the following claims, any of the claimed embodiments can be used in any combination.

In the description provided herein, numerous specific details are set forth. However, it is understood that embodiments of the invention may be practiced without these specific details. In other instances, well-known methods, structures and techniques have not been shown in detail in order not to obscure an understanding of this description.

Intense Pulsed Light (IPL) devices are utilized for various skin treatments, including hair removal, by delivering high fluence flashes to the skin. These devices are designed with a treatment window that is smaller than the tip of the device itself. Users are required to predict the size of the treatment window and move the device accordingly to ensure effective coverage. However, due to the larger size of the device tip compared to the treatment window, novice users often move the device over a greater distance than necessary. This common user error leads to reduced coverage and results in untreated areas, compromising the efficacy of the treatment. Additionally, novice users sometimes move the device not sufficiently enough thereby resulting in overexposure of skin areas.

Current IPL devices offer limited guidance to users, which often leads to suboptimal treatment results. The challenge lies in the user's ability to accurately gauge the appropriate distance to move the device between flashes. Without proper training or feedback, users may struggle to develop the necessary technique for effective use. This lack of guidance can result in inconsistent treatment, with some areas receiving excessive exposure while others remain untreated. The need for a more intuitive and user-friendly approach to IPL treatment is evident, as this would significantly enhance the user experience and treatment outcomes.

The present disclosure introduces a personal care device usage by incorporating a training mode that operates at a non-treatable flash fluence level. This training mode allows users to practice the operation of the device without the risk of skin damage. By utilizing a lower flash intensity, users can repeatedly apply the device to their skin, thereby learning the optimal technique for maximizing coverage. The training mode serves as an onboarding tool, enabling users to utilize the waiting period after a skin test-required to check for adverse reactions to different fluence levels-effectively. During this time, users can familiarize themselves with the device's operation, ensuring they are prepared to commence treatment with confidence and precision once the skin test is complete.

In an aspect of the invention, a light-based skin care device is presented, comprising a light source for treating skin, the light source is configured to operate in a plurality of light intensity modes. A processor is present and configured for selecting a light intensity mode of the light source. At least one light intensity mode of the plurality light intensity modes is a training mode for allowing users to practice operation of the system without causing harm to a user. Harm can be defined as overexposure of skin to the light source causing, for example, skin burn, irritation, an allergic reaction or any other harm that can cause discomfort to the user.

The inclusion of a training mode in the light-based skin care device allows users to practice operating the device without the risk of causing harm. This is achieved by configuring the light source to operate at a non-treatable flash fluence level, which is significantly lower than the levels used for actual treatment. This operational principle ensures that users can familiarize themselves with the device's handling and movement requirements, thereby reducing the likelihood of user error during actual treatment sessions. By providing a safe, low-intensity training mode, the device addresses the common issue of inconsistent coverage due to the user's inability to accurately predict the treatment window size. This mode allows users to practice the necessary movements and techniques, leading to more effective and comprehensive treatment coverage when the device is used at higher, treatable intensities. The training mode thus enhances the overall user experience and treatment efficacy by ensuring that users are well-prepared and confident in their ability to use the device correctly. Furthermore, the training mode can be particularly beneficial during the onboarding phase, where users are required to perform a skin sensitivity test. Instead of waiting idly for the results of this test, users can utilize the training mode to practice and perfect their technique. This dual-purpose approach not only optimizes the use of the user's time but also ensures that they are ready to begin effective treatment immediately after the skin test, thereby improving the overall efficiency and user satisfaction with the device.

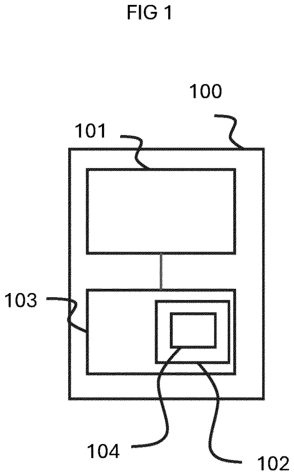

FIG 1 illustrates an embodiment. The light-based skin care device 100 includes a light source 101 that serves as the primary component for treating skin. The light source 101 is capable of emitting light at various intensities. The light source 101 operates in conjunction with a plurality of light intensity modes 102, which provide users with the flexibility to select the appropriate intensity for their specific skin type and treatment needs. The plurality of light intensity modes 102 includes a range of settings that can be adjusted to deliver the desired level of light fluence to the skin, ensuring that the treatment is both effective and safe for the user. The processor 103 plays a role in the operation of the light-based skin care device 100 by selecting the light intensity mode from the plurality of light intensity modes 102. The processor 103 receives input from the user regarding the desired treatment and, based on this input, activates the corresponding light intensity mode on the light source 101. The processor 103 is designed to manage the various operational parameters of the light-based skin care device 100, including the activation and regulation of the light intensity modes 102. This ensures that the device operates efficiently and provides the user with the optimal treatment experience. Among the plurality of light intensity modes 102, the training mode 104 stands out as a feature of the light-based skin care device 100. The training mode 104 is designed to allow users to practice the operation of the device 100 without causing harm. This mode operates at a light intensity level that is safe for the user, enabling them to become familiar with the device's functionality and handling. The training mode 104 is particularly beneficial for new users who are learning how to use the light-based skin care device 100, as the training mode 104 provides a risk-free environment for them to develop their skills and confidence before proceeding to higher intensity treatments.

According to embodiments, the device comprises an IMU sensor for providing movement information of the device, and wherein the training mode instructs the user on how to move the device for effective skin treatment.

The integration of an IMU (Inertial Measurement Unit) sensor in the device provides real-time movement information, enabling the training mode to offer precise guidance on how to move the device. This ensures that users can learn the correct motion patterns required for effective skin treatment. The advantage of providing movement information is that it significantly enhances the user's ability to perform the treatment correctly. By receiving real-time feedback on their movements, users can quickly adjust and perfect their technique, leading to more consistent and effective treatment outcomes. This reduces the learning curve and increases user confidence in using the device. Providing movement information addresses the problem of improper device movement during treatment, which can result in uneven coverage and suboptimal results. Novice users often struggle with maintaining the correct motion and speed, leading to areas of the skin being over-treated or under-treated. The IMU sensor, combined with the training mode, provides the necessary guidance to ensure that users move the device correctly, thereby solving the issue of inconsistent treatment coverage.

The IMU is a sensor that can provide data for calculating distances, for example calculated by a processor. A part of a user's skin treatment routine may be: the user moves, stops, flashes the device; thereafter again moves, stops, flashes the device. The distance between two flashes is calculated. The feedback is provided to the user if the distance (also defined as step size) is too large or too small. This feedback may be provided on a display that is part of the device or when an app on mobile device is used, on the display of the mobile device.

According to embodiments, the training mode instructs the user on how often to activate the device for effective treatment.

The technical effect of the training mode instructing the user on how often to activate the device for effective treatment is the optimization of the device's usage pattern. By providing precise instructions on the frequency of activation, the device ensures that the user applies the treatment at intervals that maximize efficacy while minimizing the risk of overexposure or underexposure to the light pulses. The primary advantage of the training mode instructing the user on how often to activate the device for effective treatment is that it enhances the overall effectiveness of the treatment. Users are guided to activate the device at the correct intervals, which helps in achieving consistent and uniform treatment results. This reduces the likelihood of user errors, such as activating the device too frequently or too infrequently, which can lead to suboptimal treatment outcomes. Additionally, this guidance can improve user confidence and satisfaction, as they are assured that they are using the device correctly. Providing a training mode instructing the user on how often to activate the device for effective treatment addresses the problem of inconsistent treatment results due to improper activation intervals. Novice users may not intuitively know how often to activate the device, leading to areas of the skin being over-treated or under-treated. By instructing the user on the optimal activation frequency, the training mode ensures that the entire treatment area receives the appropriate amount of light exposure, thereby solving the issue of uneven treatment and enhancing the overall efficacy of the device.

According to embodiments, the processor is configured to select the training mode during first time use by the user.

The processor being configured to select the training mode during first-time use by the user facilitates the automation of the initial training process. This ensures that new users are immediately guided through the training mode without needing to manually select it, thereby facilitating a smoother onboarding experience and ensuring that users are properly trained before they begin actual treatment. The primary advantage of this feature is that it enhances user convenience and ensures consistent training for all new users. By automatically initiating the training mode, the device guarantees that every user receives the necessary guidance on how to use the device effectively and safely from the outset. This reduces the likelihood of user error during initial use and increases user confidence in operating the device. This feature addresses the problem of inconsistent user training and potential user error during the initial use of the device. Novice users may not be aware of the need for training or may skip the training step if it requires manual selection. By configuring the processor to automatically select the training mode during first-time use, the device ensures that all users receive the essential training, thereby solving the issue of improper device handling and enhancing the overall efficacy and safety of the treatment.

According to embodiments, the training mode is selectable via a user interface integrated in a software application running on a device external from the light-based skin care device.

The effect of the training mode being selectable via a user interface integrated in a software application running on a device external from the light-based skin care device is the enhancement of user interaction and control over the training process. This allows users to easily access and activate the training mode through a familiar interface, such as a smartphone or tablet, providing a seamless and user-friendly experience. The primary advantage of this feature is that it increases the accessibility and convenience of the training mode. Users can activate and control the training mode through an external device, which may offer a more intuitive and comprehensive interface compared to the controls on the light-based skin care device itself. This can lead to a more engaging and effective training experience, as users can receive detailed instructions, feedback, and progress tracking through the external application. This feature addresses the problem of limited user interaction and control with the training mode on the light-based skin care device. The built-in controls on the device may be insufficient for providing a comprehensive training experience, leading to user frustration and suboptimal training outcomes. By making the training mode selectable via a user interface on an external device, the system ensures that users have a more effective and user-friendly way to access and utilize the training mode, thereby solving the issue of inadequate training and improving the overall efficacy and user satisfaction with the device.

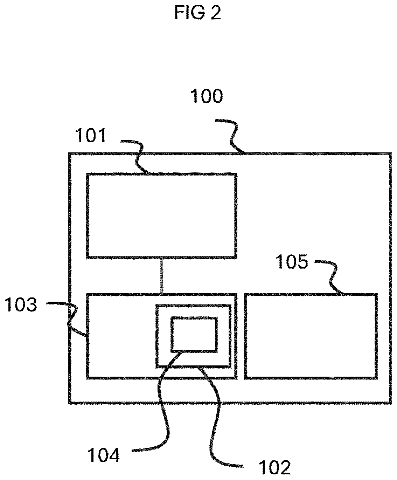

FIG 2 illustrates an embodiment. The light-based skin care device 100 includes a light source 101 that serves as the primary component for treating skin. The light source 101 is capable of emitting light at various intensities, which are necessary for different skin treatment applications. The light source 101 operates in conjunction with a plurality of light intensity modes 102, which provide users with the flexibility to select the appropriate intensity for their specific skin type and treatment needs. The plurality of light intensity modes 102 includes a range of settings that can be adjusted to deliver the desired level of light fluence to the skin, ensuring that the treatment is both effective and safe for the user. The processor 103 plays a role in the operation of the light-based skin care device 100 by selecting the light intensity mode from the plurality of light intensity modes 102. The processor 103 receives input from the user regarding the desired treatment and, based on this input, activates the corresponding light intensity mode on the light source 101. The processor 103 is designed to manage the various operational parameters of the light-based skin care device 100, including the activation and regulation of the light intensity modes 102. This ensures that the device operates efficiently and provides the user with the optimal treatment experience. Among the plurality of light intensity modes 102, the training mode 104 stands out as a feature of the light-based skin care device 100. The training mode 104 is designed to allow users to practice the operation of the device without causing harm. This mode operates at a light intensity level that is safe for the user, enabling them to become familiar with the device's functionality and handling. The training mode 104 is particularly beneficial for new users who are learning how to use the light-based skin care device 100, as the training mode 104 provides a risk-free environment for them to develop their skills and confidence before proceeding to higher intensity treatments. The user interface 105 is integrated into the light-based skin care device 100 and facilitates interaction between the user and the device. The user interface 105 allows users to select and activate the various light intensity modes 102, including the training mode 104. The user interface 105 may consist of buttons, touchscreens, or other input mechanisms that enable the user to navigate through the device's settings and choose the desired mode of operation.

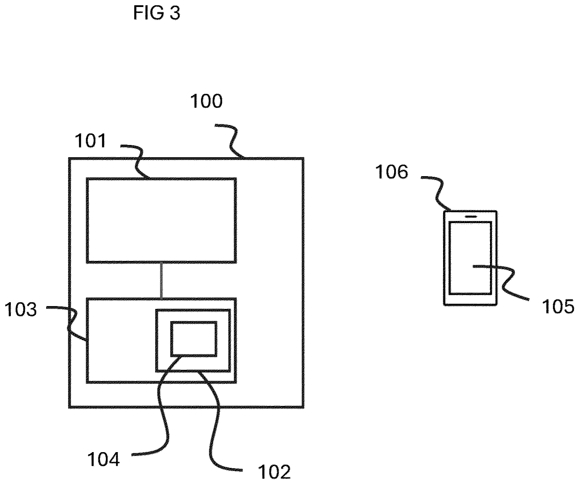

FIG 3 illustrates an embodiment. The light-based skin care device 100 includes a light source 101 that serves as the primary component for treating skin. The light source 101 is capable of emitting light at various intensities, which are necessary for different skin treatment applications. The light source 101 operates in conjunction with a plurality of light intensity modes 102, which provide users with the flexibility to select the appropriate intensity for their specific skin type and treatment needs. The plurality of light intensity modes 102 includes a range of settings that can be adjusted to deliver the desired level of light fluence to the skin, ensuring that the treatment is both effective and safe for the user. The processor 103 plays a role in the operation of the light-based skin care device 100 by selecting the light intensity mode from the plurality of light intensity modes 102. The processor 103 receives input from the user regarding the desired treatment and, based on this input, activates the corresponding light intensity mode on the light source 101. The processor 103 is designed to manage the various operational parameters of the light-based skin care device 100, including the activation and regulation of the light intensity modes 102. This ensures that the device operates efficiently and provides the user with the optimal treatment experience. Among the plurality of light intensity modes 102, the training mode 104 stands out as a feature of the light-based skin care device 100. The training mode 104 is designed to allow users to practice the operation of the device without causing harm. This mode operates at a light intensity level that is safe for the user, enabling them to become familiar with the device's functionality and handling. The training mode 104 is particularly beneficial for new users who are learning how to use the light-based skin care device 100, as the training mode 104 provides a risk-free environment for them to develop their skills and confidence before proceeding to higher intensity treatments. The user interface 105 is integrated into a software application running on a device 106 external from the light-based skin care device 100. The user interface 105 facilitates interaction between the user and the device 100, allowing users to select and activate the various light intensity modes 102, including the training mode 104. The user interface 105 may consist of buttons, touchscreens, or other input mechanisms that enable the user to navigate through the device's settings and choose the desired mode of operation. The user interface 105 is designed to be intuitive and user-friendly, ensuring that users can easily access and control the device's functions without confusion or difficulty. The external device 106, such as a smartphone or tablet, provides a familiar and convenient platform for users to interact with the light-based skin care device 100, enhancing the overall user experience.

According to embodiments, the at least one light intensity mode is a light intensity level below a predefined threshold light intensity level.

The technical effect of having at least one light intensity mode set to a level below a predefined threshold light intensity level is the ability to provide a safe, non-treatable training mode. This lower intensity level allows users to practice using the device without the risk of causing harm or skin damage, thereby facilitating a safer learning environment. The primary advantage of this feature is that it enables users to familiarize themselves with the device's operation and handling without the fear of adverse effects. This training mode helps users develop the correct technique and confidence in using the device, which can lead to more effective and consistent treatment results when using higher, treatable intensity levels. Additionally, it reduces the risk of user error during actual treatment sessions, as users are better prepared and trained. This feature addresses the problem of user inexperience and the potential for improper use of the device, which can lead to ineffective treatment or skin damage. Novice users may struggle to use the device correctly at treatable intensity levels, resulting in inconsistent coverage and suboptimal results. By providing a light intensity mode below a predefined threshold, the device offers a safe training environment where users can practice and perfect their technique, thereby solving the issue of user inexperience and enhancing the overall efficacy and safety of the treatment.

According to embodiments, the predefined threshold light intensity level is equal to 0.2 J/cm2.

The effect of setting the predefined threshold light intensity level to 0.2 J/cm2 is the establishment of a clear and specific safety limit for the training mode. This ensures that the light intensity remains below a level that could cause any skin treatment effects, thereby providing a safe environment for users to practice with the device. The primary advantage of this feature is that it provides a quantifiable and standardized safety measure for the training mode. By defining the threshold at 0.2 J/cm2, users and manufacturers have a clear guideline to follow, ensuring that the training mode is consistently safe across different devices and usage scenarios. This standardization helps in building user trust and confidence in the safety of the device during training. This feature addresses the problem of potential safety risks associated with training modes that do not have a clearly defined light intensity limit. Without a specific threshold, there is a risk that the training mode could inadvertently cause skin treatment effects or damage. By setting the predefined threshold light intensity level to 0.2 J/cm2, the device ensures that the training mode remains non-treatable and safe, thereby solving the issue of safety concerns during user training and enhancing the overall reliability and user satisfaction with the device.

## Claims

1. A light-based skin care device (100), comprising:
a light source (101) for treating skin, the light source configured to operate in a plurality light intensity modes (102);
a processor (103) configured for selecting a light intensity mode of the light source (101);
**characterized in that**:
at least one light intensity mode of the plurality light intensity modes is a training mode (104) for allowing users to practice operation of the device (100) without causing harm to a user operating the device (100).

2. The light based skin care device (100) according to claim 1, wherein the device (100) comprises an IMU sensor for providing movement information of the device, and wherein the training mode (104) instructs the user on how to move the device for effective skin treatment.

3. The light based skin care device (100) according to any of the preceding claims, wherein the training mode (104) instructs the user on how often to activate the device (100) for effective treatment.

4. A light-based skin care device (100) according to any of the preceding claims, wherein the processor (103) is configured to select the training mode (104) during first time use by the user.

5. The light based skin care device according to any of the preceding claims, wherein the training mode (104) is selectable via a user interface (105) integrated in a software application running on a device (106) external from the light based skin care device (100).

6. The light based skin care device (100) according to any of the preceding claims, wherein the light based skin care device (100) is an IPL (Intense Pulsed Light) device.

7. The light-based skin care device (100) according to any of the preceding claims, wherein the at least one light intensity mode is a light intensity level below a predefined threshold light intensity level.

8. The light-based skin care device (100) according to claim 7, wherein the predefined threshold light intensity level is 0.2 J/cm^2.
